# EUROPEAN PATENT SPECIFICATION

(11) **EP 4 626 340 B1**
(45) Date of publication and mention of the grant of the patent: **25.11.2026**
(21) Application number: 22836022.8
(22) Date of filing: 01.12.2022
(51) Int. Cl.: A61B 17/32, A61B 17/3207, A61B 18/14

(54) **CATHETERS AND SYSTEMS FOR ENDOVASCULAR TREATMENT OF A BLOOD VESSEL**
KATHETER UND SYSTEME ZUR ENDOVASKULÄREN BEHANDLUNG EINES BLUTGEFÄSSES
CATHÉTERS ET SYSTÈMES POUR LE TRAITEMENT ENDOVASCULAIRE D'UN VAISSEAU SANGUIN

(43) Date of publication of application: 08.10.2025
(73) Proprietor: TVA Medical, Inc., Franklin Lakes, NJ 07417 (US)
(72) Inventor: NGUYEN, Hoang, Franklin Lakes, NJ 07417 (US); PALMER, Alexander, Franklin Lakes, NJ 07417 (US); THOMAS, Mark, Franklin Lakes, NJ 07417 (US); CLAVIJO, Cristian, Esteban, Lehi, UT 84043 (US); KEARNEY, Hunter Thomas, Franklin Lakes, NJ 07417 (US)
(74) Representative: Hoffmann Eitle
(86) International application number: PCT/US2022/080750
(87) International publication number: WO 2024/118100

(56) References cited:
- WO-A1-2013/009886
- WO-A1-2017/124060
- WO-A1-2017/124062

## Description

### TECHNICAL FIELD

The present disclosure relates to catheters, systems, and methods for endovascular treatment of a blood vessel, and more particularly catheters, systems, and methods having electrically conductive magnetic elements. Methods described herein do not form part of the claimed invention.

### BACKGROUND

Endovascular treatments of a blood vessel may include fistula formation. A fistula is generally a passageway formed between two internal organs (e.g., blood vessels or other bodily organs). Forming a fistula between two blood vessels can have one or more beneficial functions. For example, the formation of a fistula between an artery and a vein may provide access to the vasculature for hemodialysis patients. Specifically, forming a fistula between an artery and a vein allows blood to flow quickly between the vessels while bypassing the capillaries. In other instances, a fistula may be formed between two veins to form a veno-venous fistula. Generally, arterio-venous fistula formation requires surgical dissection of a target vein, and transecting and moving the vein for surgical anastomosis to the artery. It may therefore be useful to find less invasive and reliable devices and methods for aligning blood vessels and forming a fistula between the aligned blood vessels.

WO 2017/124062 A1 discloses devices, systems, and methods for forming a fistula between two blood vessels. The systems may comprise a first catheter including a housing and an electrode having a proximal end and a distal end. The proximal end is fixed relative to the housing and the distal end is longitudinally slidable within the housing.

WO 2017/124060 A1 discloses devices, systems, and methods for adhering tubular structures, such as blood vessels. Generally, the methods may comprise advancing a first catheter comprising a first adhesion element into a first blood vessel and a second catheter comprising a second adhesion element into a second blood vessel. The vessels may be adhered together by heating tissue using the first and second adhesion elements

WO 2013/009886 A1 discloses a modular magnetic anastomosis device.

### SUMMARY

One challenging aspect of forming a fistula (endovascular treatment) between blood vessels, though other body vessels are contemplated and possible, is properly aligning and coapting catheters in adj acent blood vessels prior to fistula formation. Embodiments of the present disclosure are directed to systems, methods, and catheters for fistula formation that provide improved catheter alignment and coaptation. Improved catheter alignment and coaptation is accomplished, in part, by using magnetic elements as a conductive material, rather than providing a conductive wire through a hole formed within a magnetic element, as will be described in greater detail below. Using the magnetic element as a conductive pathway may contribute to increased magnetic strength of the magnets, which in turn may lead to improved coaptation of the catheter elements, as described in further detail herein.

In one embodiment, a catheter includes a catheter body; a magnetic element positioned along the catheter body; a first electrode wire configured to project from a working site positioned along the catheter body; and a second electrode wire. And wherein: the magnetic element includes a distal end and a proximal end; the first electrode wire includes a first electrode wire proximal end coupled to the distal end of the magnetic element; the second electrode wire includes a second electrode distal end coupled to the proximal end of magnetic element; and the magnetic element is configured to conduct electricity from the first electrode wire to the second electrode wire.

In another embodiment, a system for forming a fistula between two blood vessels includes a first catheter and a second catheter. The first catheter includes a catheter body; a magnetic element positioned along the catheter body; a first electrode wire configured to project from a working site positioned along the catheter body; and a second electrode wire. And wherein: the magnetic element includes a distal end and a proximal end; the first electrode wire includes a first electrode wire proximal end coupled to the distal end of the magnetic element; the second electrode wire includes a second electrode distal end coupled to the proximal end of magnetic element; and the magnetic element is configured to conduct electricity from the first electrode wire to the second electrode wire. And wherein: the second catheter is configured to coapt with the first catheter and receive the second electrode wire.

In yet another example, a non-claimed method of forming a fistula between a first blood vessel and a second blood vessel include advancing a first catheter into the first blood vessel; advancing a second catheter into the second blood vessel adjacent to the first blood vessel; and forming the fistula between the first blood vessel and the second blood vessel at the working site and the second working site. The first catheter includes a catheter body; a magnetic element positioned along the catheter body; a first electrode wire configured to project from a working site positioned along the catheter body; and a second electrode wire. And wherein: the magnetic element includes a distal end and a proximal end; the first electrode wire includes a first electrode wire proximal end coupled to the distal end of the magnetic element; the second electrode wire includes a second electrode distal end coupled to the proximal end of magnetic element; and the magnetic element is configured to conduct electricity from the first electrode wire to the second electrode wire. The second catheter includes a second catheter body and a second working site.

These and additional features provided by the embodiments described herein will be more fully understood in view of the following detailed description, in conjunction with the drawings. The invention is defined by independent claim 1.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. 1 schematically illustrates a system of catheters for endovascular treatment of a blood vessel, according to one or more embodiments shown and described herein;
FIG. 2 schematically illustrates a perspective view of a catheter with a conductive magnetic element, according to one or more embodiments shown and described herein.
FIG. 3A schematically illustrates a cross-sectional view of a conductive magnetic element, wherein the conductive magnetic element further includes a conductive coating, according to one or more embodiments shown and described herein;
FIG. 3B schematically illustrates a perspective view of a conductive magnetic element, wherein the conductive magnetic array further includes a thermally insulating material, according to one or more embodiments shown and described herein;
FIG. 4A schematically illustrates a cross-sectional view of a magnetic element, according to the general state of the prior art, wherein the electrode wire extends through the center of the magnetic array;
FIG. 4B schematically illustrates a cross-sectional view of a conductive magnetic element, according to one or more embodiments herein;
FIG. 5A schematically illustrates a perspective view of an electrode wire coupled to a conductive magnetic element via a plurality of wires, according to one or more embodiments shown and described herein;
FIG. 5B schematically illustrates a perspective view of an electrode wire coupled to a conductive magnetic element via a circular plate, according to one or more embodiments shown and described herein;
FIG. 5C schematically illustrates a perspective view of an electrode wire coupled to a conductive magnetic element via a rectangular plate, according to one or more embodiments shown and described herein;
FIG. 5D schematically illustrates a perspective view of an electrode wire coupled to a conductive magnetic element via a cup-type contact, according to one or more embodiments shown and described herein;
FIG. 6A schematically illustrates a perspective view of a catheter in a bent configuration, according to one or more embodiments shown and described herein.
FIG. 6B schematically illustrates a cross-sectional view of the catheter of FIG. 6A, depicting a conductive paste between one or more magnets of the magnetic element, according to one or more embodiments shown and described herein.
FIG. 7 illustrates a flow chart of a method for forming a fistula, according to one or more embodiments shown and described herein.
FIG. 8A schematically illustrates a first catheter of the system of FIG. 1 being advanced through a first blood vessel and a second catheter of the system of FIG. 1 being advanced through a second vessel, according to one or more embodiments shown and described herein;
FIG. 8B Schematically illustrates treatment of the first blood vessel and the second blood vessel with the first catheter and the second catheter of FIG. 8A, according to one or more embodiments shown and described herein; and
FIG. 8C schematically illustrates a fistula formed between the first blood vessel and the second blood vessel, according to one or more embodiments shown and described herein.

Reference will now be made in greater detail to various embodiments of the present disclosure, some embodiments of which are illustrated in the accompanying drawings. Whenever possible, the same reference numerals will be used throughout the drawings to refer to the same or similar parts.

### DETAILED DESCRIPTION

Embodiments described herein are directed to devices, systems, and methods for endovascular treatment of a blood vessel such as, but not limited to forming a fistula, wire crossing procedures, bypass procedures, etc. For example, a catheter may be placed in each of two adjacent blood vessels to form a fistula therebetween with the catheters. However, flexibility of the catheters, spacing of vessels, thickness of the vessel walls, the tortuous anatomy of the vessels, or combinations thereof may make it difficult to provide sufficient coaptation and/or alignment between vessels for fistula formation. Embodiments of the present disclosure provide improved alignment and coaptation thereby providing improved, reliable treatment.

For example, in embodiments, a catheter for endovascular treatment of a blood vessel may generally include a catheter body, a magnetic element positioned along the catheter body, a first electrode wire configured to project from a working site positioned along the catheter body, and a second electrode wire. The magnetic element includes a distal end and a proximal end and a first electrode wire proximal end of the first electrode wire is coupled to the distal end of the magnetic element. Similarly, a second electrode distal end of the second electrode wire is coupled to the proximal end of magnetic element. The magnetic element may therefore act as a conductive element or pathway to conduct electricity from the first electrode wire to the second electrode wire, without need for passages formed through the magnetic element itself, thereby substantially maintaining magnetic strength and/or integrity. For example, if a wire were instead tunneled through the magnetic element, removal of magnetic material may lead to a weaker magnetic coupling as will be described in greater detail below. Accordingly, embodiments of the present disclosure may result in improved magnetic strength of the magnetic element (e.g., up to 25% greater or more as compared to a magnetic element that has been tunneled through for wire passage), and thereby improve alignment and coaptation between the first and second catheters. These and additional features and benefits will be described in greater detail herein.

As used herein, the term "proximal" means closer to or in the direction of an origin of an element, such as a catheter. The origin of a catheter may be a handle or other user-manipulated portion of the catheter. The term "distal" means further from the origin, or handle, of the catheter. Put another way, the term "distal" means closer to or in the direction of a tip of a catheter, which is separated from a handle or other user-manipulated portion of the catheter by the length of the catheter body.

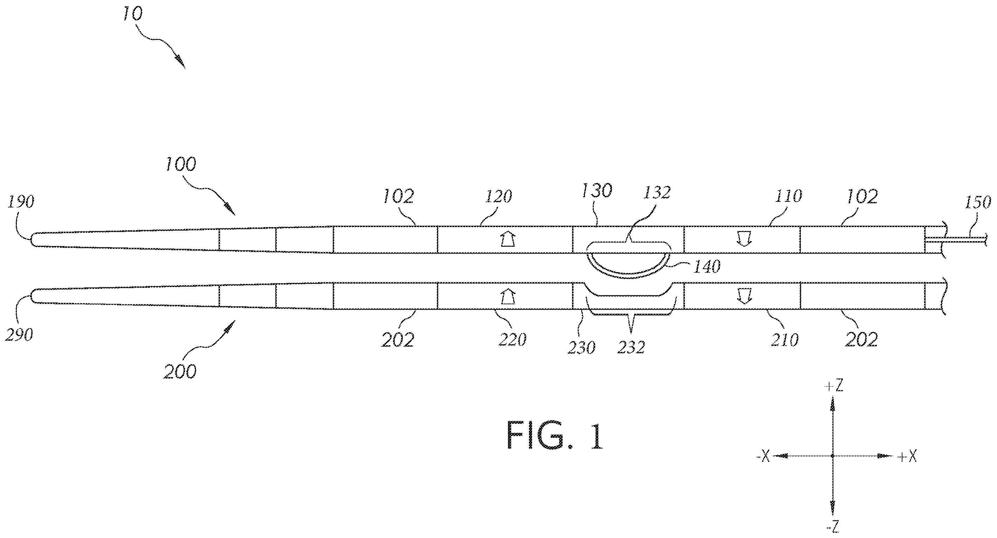

Referring now to FIG. 1, a system 10 for providing endovascular treatment of a blood vessel, such as fistula formation, is schematically illustrated. The system 10 may include a first catheter 100 and a second catheter 200. It may be appreciated that the first catheter 100 and the second catheter 200 are substantially similar to one another. Accordingly, description of the first catheter 100 generally applies to the second catheter 200 unless otherwise noted or apparent. It is noted that the first catheter 100 and the second catheter 200 may be provided within a kit and/or separately from one another.

The first catheter 100 may include a catheter body 102, a magnetic element 110, a treatment portion 130 including a first electrode wire 140, and a second electrode wire 150. It is noted that the first catheter 100 may include a greater or fewer number of components without departing from the scope of the present disclosure. The catheter body 102 may be sized to be advanced through a blood vessel and may include a distal tip 190 that is may be shaped and/or sized to aid in advancement of the first catheter 100 through a blood vessel. For example, the distal tip 190 may be pointed, tapered, and/or atraumatic for advancement through a blood vessel. The catheter body 102 may have any cross-sectional shape and any diameter suitable for intravascular use. The catheter body 102 may be formed of any material or combination of materials able to be traversed through a vasculature of a body. For example, the catheter body 102 may include, silicone, rubber, etc.

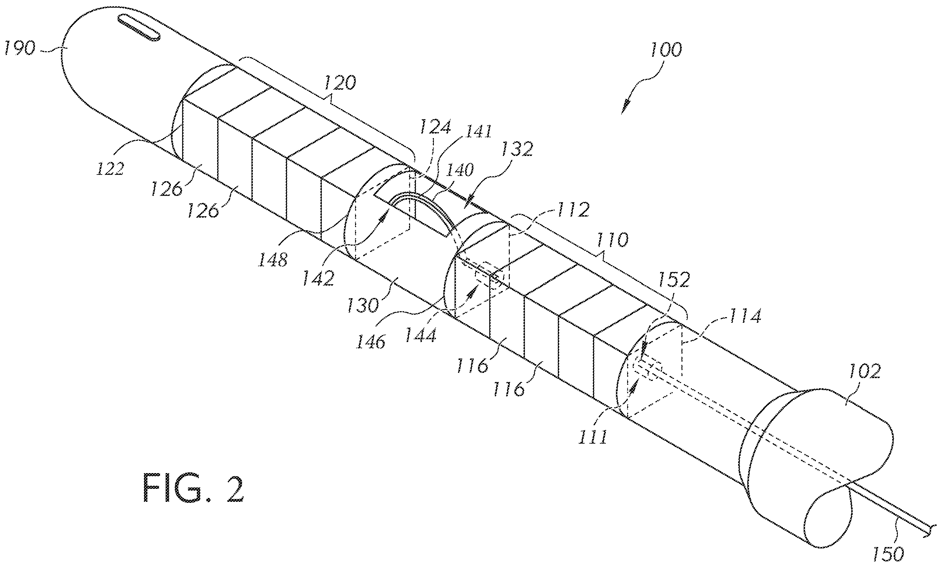

Referring now to FIGS. 1 and 2, and as noted above, the first catheter 100 may have a treatment portion 130 for endovascular treatment of a blood vessel. In embodiments, the treatment portion 130 may define a working site 132 including a fistula-forming element, which is also referred to as the first electrode wire 140. The first electrode wire 140 may extend between a treatment portion distal end 148 and a treatment portion proximal end 146 of the treatment portion 130. In embodiments, the first electrode wire 140 may have an exposed ablation surface 141 for ablating tissue. The first electrode wire 140 may be coupled to a power source (not shown), such as through the first magnetic element 110 and second electrode wire 150, as explained in further detail herein. For example, the second electrode wire 150 may be a lead wire or other conductor coupled to a power source. When activated, current may be supplied to and/or carried from tissue and fluid via the exposed ablation surface 141 of the first electrode wire 140 to facilitate ablation or vaporization of tissue to form a fistula. In embodiments, the first electrode wire 140 and/or the second electrode wire 150 may be formed of any electrically conductive material such as, but not limited to copper, steel, aluminum, gold, iron, nickel, Nitinol etc., or alloys thereof.

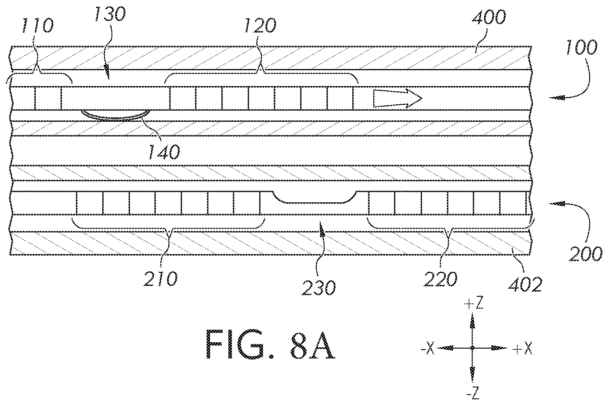

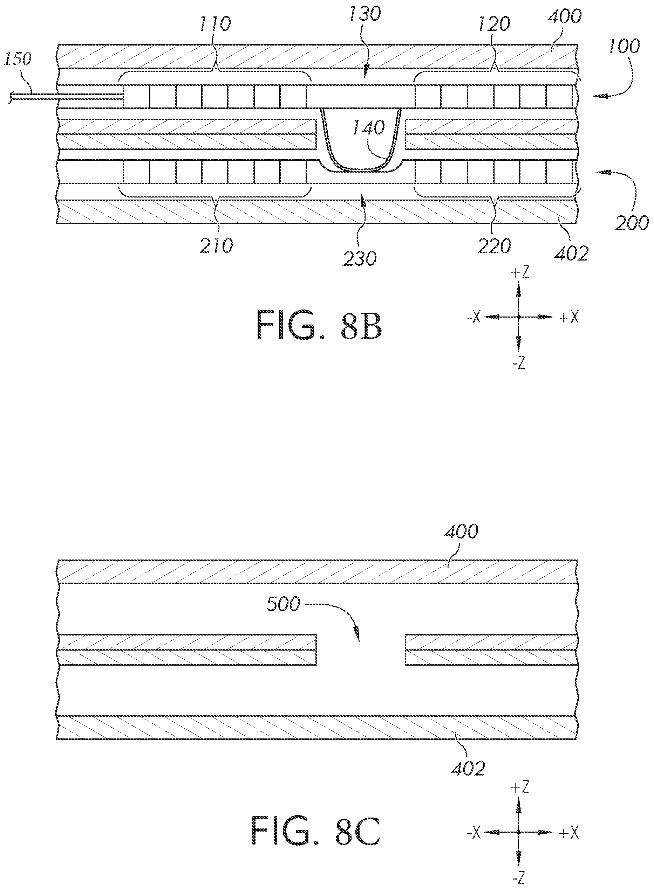

In some embodiments, the first electrode wire 140 may be a spring wire or leaf spring electrode, which may be movable between a retracted configuration (see FIG. 8A), in which the first electrode wire 140 is retained within the first catheter 100, and a protruding configuration (see FIG. 8B), in which the first electrode wire 140 projects beyond the outer surface of the catheter body 102.

As illustrated in FIG. 1, the first electrode wire 140 may be arc shaped, though other shapes are contemplated and possible (e.g., rectangular, square, angular, etc.). The size and shape of the first electrode wire 140 may be varied based on factors including tissue thickness and density, as well as desired fistula size, shape, and location.

Referring to FIG. 2, and as previously stated, the first catheter 100 may include a magnetic element 110. The first catheter may also include a second magnetic element 120. In this manner, the magnetic element 110 may also be referred to herein as a "first magnetic element". The first and second magnetic elements 110, 120 may be positioned along the catheter body 102, and may each include a distal end and proximal end. For example, and in embodiments, the first magnetic element 110 may include a first magnetic element distal end 112 and a first magnetic element proximal end 114. Similarly, the second magnetic element 120 may include a second magnetic element distal end 122 and a second magnetic element proximal end 124. As described in further detail herein, the first and/or second magnetic elements 110, 120 may be configured to conduct electricity. In other words, the first and/or second magnetic elements 110, 120 may include electrically conductive elements or materials that allow an electric current to be transmitted from the proximal end to the distal end, or vice versa, of the magnetic element. For example, the first and second magnetic elements 110, 120 may be formed of any magnetic or ferromagnetic and electrically conductive material, as explained in further detail below, and may include any number of magnets. In some embodiments, only the first magnetic element 110 may be electrically conductive while the second magnetic element 120 is not electrically conductive or is not electrically coupled to the first electrode wire 140.

For example, in embodiments, the first and/or second magnetic elements 110 and 120 may include an array of magnets arranged longitudinally along the length of the catheter 100, such as illustrated in FIG. 2. The array of magnets may include a plurality of magnets 116 and 126. Each of the magnets of the plurality in the first and second magnetic elements 110, 120 may have substantially the same dimensions (e.g., height, width, and depth) and substantially the same magnetic strength. In some embodiments, the plurality of magnets may have a combination of different sized magnets, different shaped magnets, and/or magnets of differing magnetic strength. For example, the individual magnets of the plurality of magnets 116 and 126 may have a substantially cubic shape, circular shape, oval shape, or any shape for traversal within a targeted blood vessel. The array of magnets may include any number of magnets 116, 126 such as 2 or more, 5 or more, 6 or more, 10 or more, 18 or more, etc.

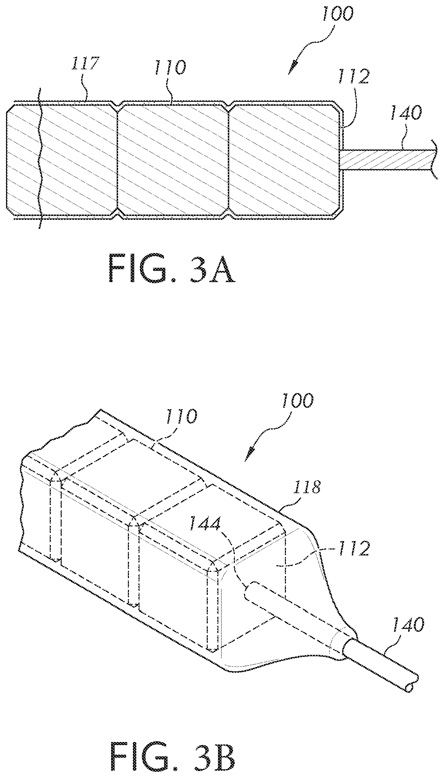

Now referring to FIGS. 3A and 3B, the first and/or second magnetic elements 110, 120 may be electrically conductive through a conductive coating 117, or by being formed of one or more magnetic materials, such as but not limited to alloys of rare earth elements (e.g., samarium-cobalt magnets or neodymium magnets, such as N52 magnets) or alnico. In some variations, the first and/or second magnet elements 110, 120 may include anisotropic magnets; in other variations, the first and/or second magnet elements 110, 120 may include isotropic magnetics. In some variations, the first and/or second magnet elements 110, 120 may be formed from compressed powder. In some variations, a portion of the first and/or second magnet elements 110, 120 may comprise one or more soft magnetic materials, such as but not limited to iron, cobalt, nickel, ferrite, nickel-plated copper, or alnico (Al-Ni-Co).

Now referring to FIG. 3B, and in embodiments, the catheter 100 may further include a thermally insulating material 118. As illustrated in FIG. 3B, the thermally insulating material 118 may overlay or coat the one or more magnetic elements 110 and 120, the first or second electrode wire 140 and 150, or combinations thereof. The thermally insulating material 118 may also be combined with the conductive coating 117, such that the thermally insulating material 118 may overlay or provide an additional coating over the conductive coating 117 (not shown). The thermally insulating material 118 may thereby insulate the catheter body from the electrically conductive magnetic elements. This may also operate to prevent inadvertent thermal ablation of the blood vessel at the one or more magnetic elements 110, 120, as the one or more magnetic elements 110, 120 may increase in temperature as current is applied. The thermally insulating material 118 may include polytetrafluoroethylene (PTFE), although other thermally insulating materials are contemplated. The thermally insulating material 118 may be the same material that makes up the catheter body 102. In some embodiments, the thermally insulting material 118 may be instead or in addition to being thermally insulative may be electrically insulative.

In some embodiments, in addition to the thermally insulating material 118 or in lieu of the thermally insulating material 118 the selection of a particular magnetic metal alloy for the one or more magnetic elements 110 and 120, or for the conductive coating 117, may limit the need for thermally insulating materials on the one or more magnetic elements 110, 120. For example, the choice of Neodymium (Nd-Fe-B) magnets, Alnico (Al-Ni-Co) magnets, or the like as the magnetic metal alloy may limit the increase in surface temperature experienced by the one or more magnetic elements 110, 120 when the current is applied.

In embodiments, the first and second magnetic elements 110 and 120 may be positioned on either side of the first treatment portion 130. For example, and as shown in FIG. 2, the first magnetic element distal end 112 may be in contact with or positioned close to the proximal end 146 of the first treatment portion 130. Similarly, the second magnetic element proximal end 124 may be in contact with or positioned closely to the distal end 146 of the first treatment portion 130.

Referring back to FIG. 2, the first electrode wire 140 may include a first electrode wire distal end 142 and a first electrode wire proximal end 144. Similarly, the second electrode wire 150 may include a second electrode wire distal end 152 and a second electrode wire proximal end (not shown). The first electrode wire proximal end 144 may be coupled to the first magnetic element distal end 112. The first electrode wire distal end 142 may be coupled to the second magnetic element proximal end 124 or it may be freely translatable toward and away from the second magnetic element 120 such that the first electrode wire may transition between an extended configuration or a low-profile confirmation, as described above.

The second electrode wire distal end 152 of the second electrode wire 150 is coupled to the first magnetic element proximal end 114. As previously stated, the second electrode wire proximal end may be electrically coupled to a power source (not shown), such that current may be supplied through the second electrode wire. When activated, current may be supplied to the second electrode wire 150 from the power source, which is thereby conducted to the first magnetic element 110. As the first magnetic element 110 is electrically conductive as described above, current is conducted from the second electrode wire 150 through the first magnetic element to the first electrode wire 140. As previously stated, the first electrode wire 140 may then carry the current to tissue and fluid via the exposed ablation surface 141 to facilitate ablation or vaporization of tissue to form a fistula, for example.

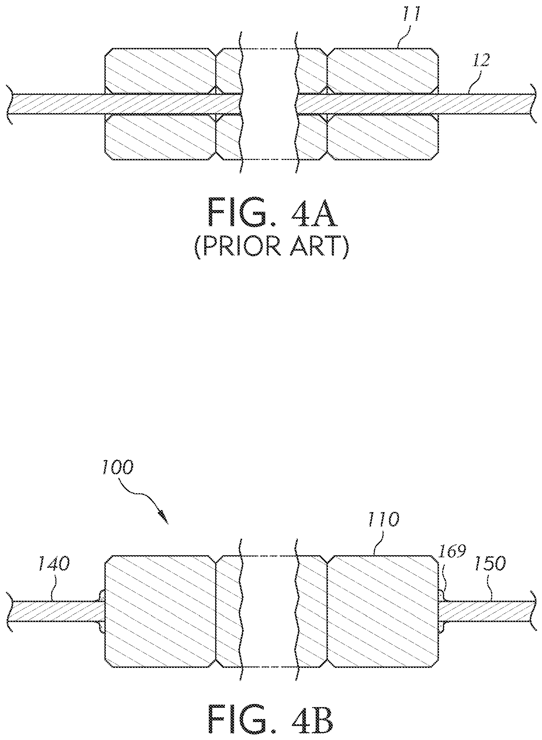

Now referring to FIGS. 4A and 4B, FIG 4A illustrates a cross-section of a catheter where a wire 12 is tunneled through a magnetic element 11. Accordingly, magnetic material is removed from the magnetic element 11, causing a reduction in magnetic strength. FIG. 4B comparatively illustrates a cross-section of a catheter according to embodiments herein, wherein the electrode wire does not extend or tunnel through the magnetic element (e.g. the magnetic elements are solid). Instead, the magnetic element 110 is used as a conductor to provide current from the second electrode wire 150 to the first electrode wire 140. Accordingly, a magnetic element 110 according to the present disclosure may have greater magnetic strength providing improved coaptation than the assembly of FIG. 4A. The additional magnetic strength may be up to 25% greater than where the electrode wire tunnels through the magnetic element.

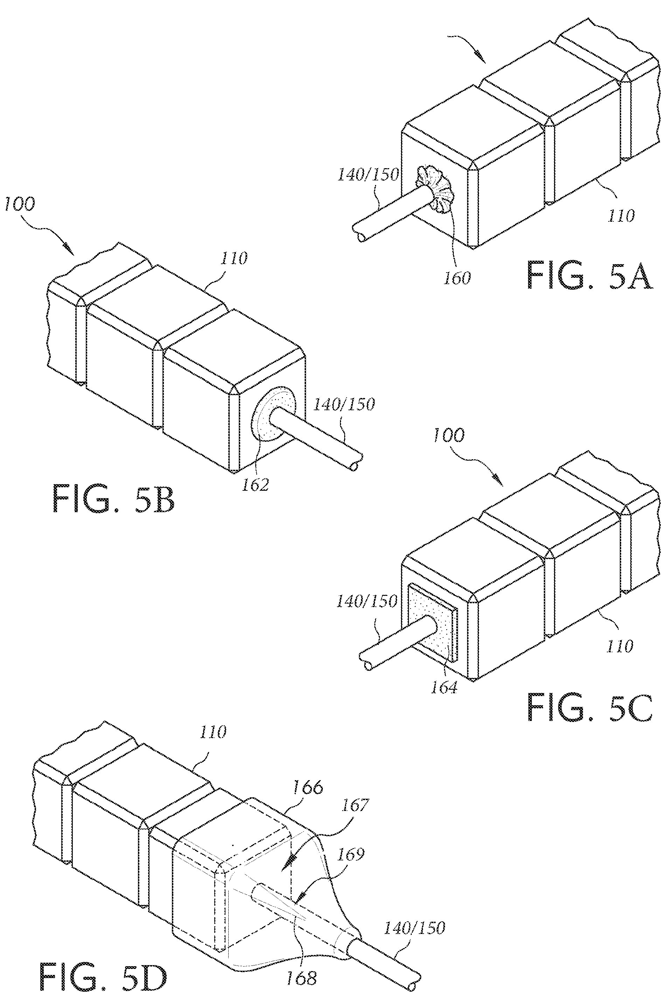

Each of the first electrode wire and the second electrode wire may be coupled to a respective side of a magnetic element (e.g., magnetic element 110) via any connection technique suitable to allow flow of current from the second electrode wire 150, through the magnetic element, such as magnetic element 110, to the first electrode wire 140. For example, the electrode wires, may be adhered, brazed, soldered, welded or otherwise connective to a respective surface of the magnetic element sufficient to allow for current to flow therethrough. Referring to FIGS. 5A to 5D various non-limiting coupling techniques are illustrated. In embodiments including connection via adhesives, the adhesives may include silicone adhesives, acrylic adhesives, epoxy adhesives, low-viscosity adhesives, or combinations thereof. The adhesives may also be electrically conductive, such as through the addition of graphite particles suspended within the adhesive. The electrically conductive adhesives may include LOCTITE's^{®} ABLESTIK line, including but not limited to: 84-1LMISR4, CA 3556HF, CE 3103WLV, ICP 4000, or combinations thereof. This may operate to form an electrically conductive coupling between the electrode and magnetic element.

In some embodiments, the ends of the first electrode wire 140 and/or the second electrode wire 150 may be structured or include structure to aid in coupling the electrode wire to the respective magnetic element 110 and/or 120. For example, and with reference to FIG. 5A an end of an electrode wire 140 and/or 150 connected to the magnetic element 110, may thereby provide a frayed connector 160 having a plurality of contacting wires. Each of the plurality of contacting wires may be coupled to a surface of the magnetic element 110. Thereby, the fraying of the end may increase the surface area of the connection and make for a stronger bond, decrease the risk of the electrode wire shorting during use, or both. For example, the first electrode wire proximal end 144 may be frayed to provide the frayed connector 160, which may then be coupled to the first magnetic element distal end 112. Similarly, the second electrode wire distal end 154 may be frayed such that the second electrode wire distal end 154 has the plurality of contacting wires, and each of the plurality of contacting wires may be coupled to the first magnetic element proximal end 114. It is noted the FIGS. 5A-5D include reference number 140/150 to indicate that the illustration may depict either end of the magnetic element 110 with either the first electrode wire 140 or the second electrode wire 150 for simplicity. In embodiments, the frayed connector 160 may be combined with a magnetic element 110 having the conductive coating 117 and/or the thermally insulating material 118. In other words, the conductive coating 117 and/or the thermally insulating material may overlay or coat the frayed connector 160.

Referring now to FIGS. 5B and 5C, coupled to the end of the electrode wire 140 and/or 150 may be a conductive plate or contact. The conductive plates may be any geometric shape, such as, but not limited to a circular-shaped conductive plate 162, such as illustrated in FIG. 5B and rectangular-shaped conductive plate 164, such as illustrated in FIG. 5C, though other polygonal or non-polygonal shapes are contemplated and possible. The conductive plate 162/164 may be coupled to the end of the particular electrode wire 140 and/or 150 via welding, adhesive, brazing, soldering, or the like. For example, an end of the electrode wire may be coupled to a center of the conductive plate 162/164. For example, similar to the frayed connector 160 described above, the first electrode wire proximal end 144 may include a conductive plate (e.g., 162 or 164) which is coupled to the first magnetic element distal end 112. The conductive plate 162/164 may include a coupling surface, such as a flat coupling surface configured to abut a surface of the magnetic element 110. Similarly, the second electrode wire distal end 152 may include the conductive plates 162 and 164, wherein the conductive plates may be coupled to the first magnetic element proximal end 114. It is contemplated that the conductive plate may aid in spreading the electrical current in a radial direction and may thereby reduce stress on the connection between the magnetic element and electrode wire when the catheter is in operation. In embodiments, the conductive plate 162 or 164 may be formed of any electrically conductive material such as, but not limited to copper, steel, aluminum, gold, iron, nickel, etc. In embodiments, the conductive plates 162 and 164 may be combined with a magnetic element 110 having the conductive coating 117 and/or the thermally insulating material 118. In other words, the conductive coating 117 and/or the thermally insulating material may overlay or coat the conductive plates 162 and 164.

Now referring to FIG. 5D, in some embodiments the electrode wires 140 and/or 150 may be coupled to the one or more magnetic elements using a cup-type connection 166. As illustrated in FIG. 5D, the end of the electrode wire 140 or 150 may include the cup-type connection 166, which may "nest" on top of the magnetic element 110. The cup-type connection 166 may stay coupled to the magnetic element 110 through the use of the adhesive, an interference fit, or combinations thereof. The adhesive may also be electrically conductive, as previously noted. The cup-type connection 166 may define a first recess 167 sized and shaped to receive the first magnetic element proximal end 114, the first magnetic element distal end 112, the second magnetic element proximal end 124, or combinations thereof. The cup-type connection may also define a second recess 169 sized and shaped to accept one or more prongs 168 connected to, and extending from, the magnetic element 110. The one or more prongs 168 may alternatively be connected to, and extend from, the cup-type connection 166 to which the magnetic element 110 may define the recess sized and shaped to accept the one or more prongs 168. The one or more prongs 168 may also be electrically conductive, such that the one or more prongs 168 conducts current from the first or second electrode wires 140 and 150 to the one or more magnetic elements 110 and 120. The one or more prongs 168 may also be magnetic, such that the one or more prongs 168 magnetically coapt to the end of the particular electrode wire 140/150 within the second recess 169. In some embodiments, there may not be one or more prongs. Instead, the particular electrode wire 140 or 150 may extend through the second recess to contact the magnetic element 110 or 120 and be held thereto by the cup-type connection 166. The cup-type connection 166 may be formed of any suitable material and may or may not be electrically conductive. For example, the cup-type connection 16 may be formed of silicon or plastic material such as or similar to the thermally insulating material 118. Alternatively, the cup-type connection 166 may be electrically conductive and formed of any electrically conductive material such as those disclosed herein. In embodiments, the cup-type connection 166 may be combined with a magnetic element 110 having the conductive coating 117 and/or the thermally insulating material 118. In other words, the conductive coating 117 and/or the thermally insulating material may overlay or coat the cup-type connection 166.

Referring back to FIG. 2, in some embodiments the electrode wires 140 and 150 may also be coupled to the magnetic element 110 and/or 120 by being partially embedded in the magnetic elements 110 and 120. Similar to the cup-type connection 166, the magnetic elements 110 and 120 may define a recess 111 within that may be sized and shaped to receive the electrode wire 140 or 150. The electrode wire 140 or 150 may then stay coupled to the magnetic element 110 or 120 through adhesives, an interference fit, or any other connection known to one of ordinary skill in the art, such as through the methods mentioned immediately below.

Referring back to FIG. 1, similar to the first catheter 100, the second catheter 200 generally includes a catheter body 202, a magnetic element 120, and a second treatment portion 230, which may further include a second working site 232. It is noted that the second catheter 200 may include a greater or fewer number of components without departing from the scope of the present disclosure.

The catheter body 202 may include a distal tip 290 that may be shaped and/or sized to aid in advancement of the second catheter 200 through a blood vessel. For example, the distal tip 290 may be pointed, tapered, and/or atraumatic for advancement through a blood vessel. The catheter body 202 may have any cross-sectional shape and any diameter suitable for intravascular use. The catheter body 202 may be formed of any material or combination of materials able to be traversed through a vasculature of a body. For example, the catheter body 202 may include, silicone, rubber, etc.

As noted above, the second catheter 200 may define a second treatment portion 230 that may be configured to be aligned or coapted with the treatment portion 130 of the first catheter 100 such as when the first catheter 100 and the second catheter 200 are aligned in adjacent vessels. The second treatment portion 230 may define a second working site 232, which may be configured to receive the first electrode wire 140 as it passes through tissue of the adjacent vessels to form a fistula. The second working site 232 may have a shape (e.g., a concave portion) that corresponds to and is complementary (e.g., inverse, reciprocal) to the first electrode wire 140 to match and conform to the first electrode wire 140 when the treatment portions 130 and 230 are aligned and/or coapted. The second working site 232 may be electrically conductive and/or electrically insulative. For example, where electrically conductive, the second working site 232 may act as an extension of the first electrode wire 140 to aid in ablation or removal of tissue. Where electrically insulative, the second working site 232 may insulate portions of the body and/or second catheter 200 from electrical contact by the first electrode wire 140. For example, the second working site 232 may include a backstop (e.g., a ceramic or other insulative backstop).

Also similar to the first catheter, the second catheter 200 may further include one or more magnetic elements for alignment or coaptation purposes of the first catheter 100 to the second catheter 200. The one or more magnetic elements may be substantially identical to the first and second magnetic elements for the first catheter 100, with the exception that the polarity may be different to align or coapt the treatment portion 130 with the second treatment portion 230 and/or direct the working site 132, also referred to herein as the "first working site," toward the second working site 232. In embodiments, the second catheter 200 may include a first magnetic element 210 and a second magnetic element 220. Each of the first and second magnetic elements 210, 210 may include a plurality of magnets arranged in an array. For example, the first and second magnetic elements may include a similar number of magnets as described above with respect to the first catheter 100. It is noted that the first and second magnetic elements 210, 220 may be made of the same or different materials as noted above with respect to the first catheter 100.

It should be appreciated that in systems comprising two catheters, either the first catheter 100 or the second catheter 200 may comprise ferromagnetic elements (i.e., elements attracted to but not generating a permanent magnetic field). For example, in some variations, the first catheter 100 may include one or more ferromagnetic elements while the second catheter 200 may comprise one or more permanent magnets. In other variations, the second catheter 200 may include one or more ferromagnetic elements while the first catheter 100 may comprise one or more permanent magnets. However, in other variations, one or both of the first catheter 100 and the second catheter 200 may include any suitable combination of ferromagnetic, permanent, and/or other suitable kinds of magnets.

While shown in FIG. 1 as being configured to receive the first electrode wire 140, i.e., having a complementary shape, it should also be appreciated that in some variations, the second treatment portion 230 of the second catheter 200 may not be configured to receive the first electrode wire 140. In some variations, the second treatment portion 230 of the second catheter 200 may include a third electrode wire (not shown), such as described hereinabove with respect to the first electrode wire 140, in addition to or instead of the first catheter 100 having the first electrode wire 140. In these embodiments, the second catheter 200 may further include a fourth electrode wire. Accordingly, in some embodiments, the first and second catheters 100 and 200 may be substantially identical to one another.

It is noted that the catheters 100, 200 and any accompanying elements may be sized and/or shaped to be advanced through any target blood vessel. For example, blood vessels having an internal diameter of about 3 mm, it may be desirable to configure any of the catheter elements to be less than about 3 mm in diameter/width. In some embodiments, the catheters 100, 200 may have any suitable diameter for intravascular use, such as, for example, about 4 French (1.33 mm), about 5.7 French (1.9 mm), about 6.1 French (6.03 mm), about 7 French (2.33 mm), about 8.3 French (2.77 mm), or a value between about 4 French (1.33 mm) and about 9 French (3.0 mm), between about 4 French (1.33 mm) and about 7 French (2.33 mm), between about 4 French (1.33 mm) and about 6 French (2.0 mm), or the like.

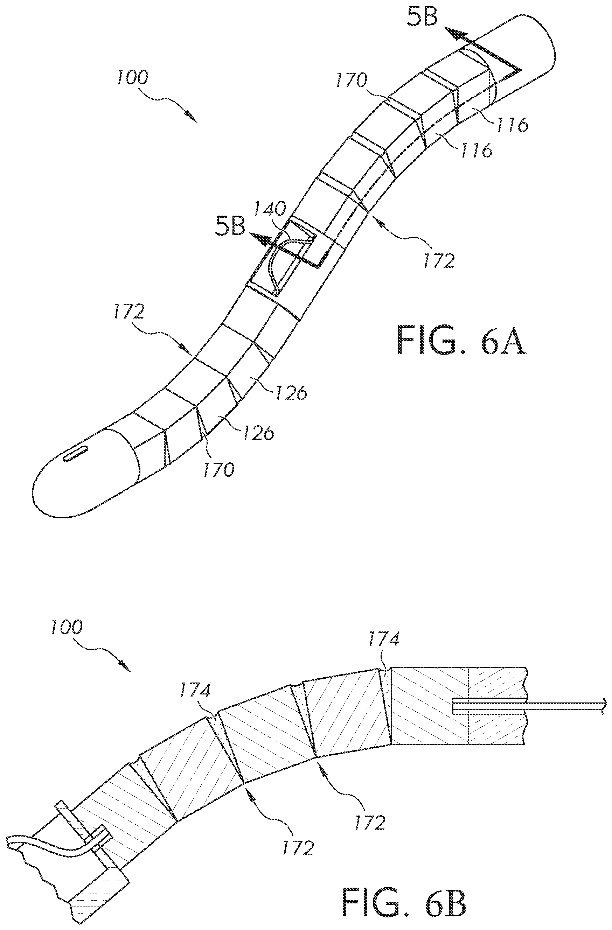

Now referring to FIGS. 6A, and in embodiments, catheters herein may be flexibly bent or transformed such as to allow the catheter to more easily traverse a subject's tortuous anatomy. However, during bending, gaps 170 may appear or be present between adjacent magnets within the plurality of magnets 116 or 126. The gaps 170 may also cause adjacent magnets within the plurality of magnets 116 or 126 to only contact each other at discrete contact points 172. Such gaps 170 may reduce contact for electrical conduction. The discrete contact points 172 may lead to a channeling of current between the individual magnets of the plurality of magnets 116 or 126, which may thereby lead to an increase in temperature. This increase in temperature may lead to an overheating of the catheter body 102 or the plurality of magnets 116 or 126, potentially damaging the plurality of magnets 116 or 126, or the catheter body 102 (e.g. by potentially melting the catheter body). Accordingly, in some embodiments, an electrically conductive paste 174 may be situated between each adjacent magnet of the plurality of magnets 116 or 126 to maintain electrical conductivity through the plurality of magnets 116 or 126. In other words, the catheters 100 and 200 herein may include the electrically conductive paste 174 interposed between one or more of the plurality of magnets 116 or 126 of the magnetic elements 110 and 120. The electrically conductive paste 174 may thereby operate to maintain electrical conductivity through the plurality of magnets 116 or 126 and the magnetic elements 110 and 120, even if the catheters herein or flexibly bent or transformed. In embodiments, the electrically conductive paste 174 may be flexible and may include, but may not be limited to, silicone with suspended graphite particles, tungsten disulfide, carbon conductive grease, or combinations thereof.

FIG. 6B illustrates a cross-section of a length of the catheter of FIG. 6A. As illustrated in FIG. 6B, the electrically conductive paste 174 may operate to maintain electric conductivity between the entirety of the faces of the individual magnets of the plurality of magnets 116 or 126 even when the catheter is flexibly bent or transformed. It is contemplated that the inclusion of the electrically conductive paste 174 may prevent the preferential channeling of current between each of the plurality of magnets 116 or 126 through the discrete contact points 172, and may thereby prevent damage occurring to the plurality of magnets 116 or 126 or to the catheter body 102.

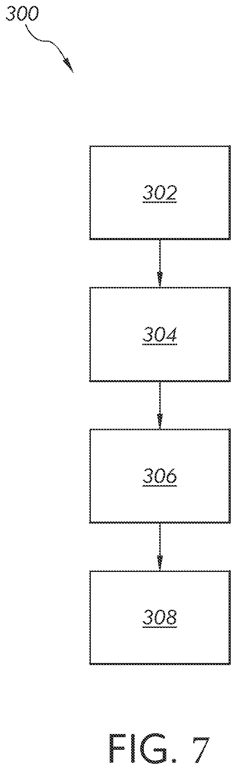

Referring now to FIG. 7, a flow chart illustrating a method 300 of forming a fistula is generally illustrated. It is noted that the method 300 may include a greater or fewer number of steps, taken in any order, without departing from the scope of the present disclosure. It is noted that the method 300 illustrated in FIG. 7 may be best understood when reviewed in conjunction with FIGS. 8A-8C, which generally illustrate alignment and treatment of a blood vessel using the first and second catheters 100, 200 as described herein.

Still referring to FIG. 7, at block 302 the method 300 includes advancing the first catheter 100 through a first blood vessel 400 as illustrated in FIG. 8A. For example, a user may advance the first catheter 100 through the first blood vessel 400 to a first treatment location so that the treatment portion 130 is advanced to the first treatment location.

Referring again to FIG. 7, at block 304 the method 300 further includes advancing the second catheter 200 through a second blood vessel 402. For example, and as illustrated in FIG. 8A, the user may advance the second catheter 200 through the second blood vessel 402 to a second treatment location in the second vessel. It is noted that the first blood vessel 400 and the second blood vessel 402 may be adjacent vessels such as a vein and an artery, though vein to vein and artery to artery treatments are contemplated and possible.

At block 306 the method 300 may include aligning the treatment portion 130 of the first catheter 100 with the treatment portion 230 of the second catheter 200. As shown in FIG. 8B, as the second catheter approaches the treatment location, and as the first and second treatment portions 130 and 230 are substantially aligned (e.g., both longitudinally and rotationally), the first and second treatment portions 130 and 230 are drawn to one another by the interaction of the attraction of the magnetic elements of the first and second catheters.

The magnetic attraction may be sufficient to draw the first blood vessel 400 into contact with the second blood vessel 402. For example, and as illustrated in FIG. 8B, magnetic attraction may cause the first catheter 100 and the second catheter 200 to magnetically mate and pull together, thereby pulling the first blood vessel 400 and the second blood vessel 402 into contact with one another. Accordingly, once aligned, the first catheter 100 and the second catheter 200 may coapt together and sandwich tissue for the first vessel and the second vessel therebetween. In some embodiments, the method 300 may further include confirming alignment of the first catheter 100 and the second catheter 200 via fluoroscopy or other imaging techniques.

Referring again to FIG. 7, at block 308 the method 300 may further include, after alignment between the treatment portions 130, 230 of the first catheter 100 and the second catheter 200, performing a vascular treatment procedure to modify the first blood vessel 400 and/or the second blood vessel 402 at the first treatment location and/or the second treatment location. For example, the first electrode wire 140 of the treatment portion 130 may be activated by providing a supply of energy to the first electrode wire 140 from an energy source (e.g., an RF generator or the like). In particular, the supply of energy may travel from the energy source through the second electrode wire 150 and the magnetic element 110, before passing to the first electrode wire 140. Activation of the first electrode wire 140 may ablate tissue of the first blood vessel 400 and the second blood vessel 402 to form a fistula 500 between the first blood vessel 400 and the second blood vessel 402, as generally illustrated in FIG. 8C. Upon formation of the fistula 500, the first catheter 100 and the second catheter 200 may be withdrawn from the respective first blood vessel 400 and the second blood vessel 402.

It should now be understood that embodiments of the present disclosure are directed to devices, systems, and methods for forming a fistula between two blood vessels. In particular, devices, systems, and methods for improved catheter alignment and coaptation by using magnetic elements as a conductive material, rather than providing a conductive wire through a hole formed within a magnetic element. Using the magnetic element as a conductive pathway may contribute to increased magnetic strength of the magnets, which in turn may lead to improved coaptation of magnetic elements of separate catheters, and thereby improved fistula creation.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

While particular embodiments have been illustrated and described herein, it should be understood that various other changes and modifications may be made without departing from the scope of the claimed subject matter. It is therefore intended that the appended claims cover all such changes and modifications that are within the scope of the claimed subject matter.

## Claims

1. A catheter (100), comprising:
a catheter body (102);
a magnetic element (110) positioned along the catheter body (102), the magnetic element (110) comprising a distal end (112) and a proximal end (114);
a first electrode wire (140) configured to project from a working site (132) positioned along the catheter body (102), the first electrode wire (140) comprising a first electrode wire proximal end (144); and
a second electrode wire (150) comprising a second electrode distal end (152), wherein:
the first electrode wire proximal end (144) is coupled to the distal end (112) of the magnetic element (110);
the second electrode wire distal end (152) is coupled to the proximal end (114) of magnetic element (110);
**characterized in that**
the magnetic element (110) is configured to conduct electricity from the first electrode wire (140) to the second electrode wire (150); and
the first electrode wire (140) is coupled to the distal end (112) of the magnetic element (110) and the second electrode wire distal end (152) is coupled to the proximal end (114) of magnetic element (110) to allow flow of current from the second electrode wire (150), through the magnetic element (110), to the first electrode wire (140).

2. The catheter (100) of claim 1, wherein the magnetic element (110) comprises an array of magnets.

3. The catheter (100) of claim 2, wherein:
the array of magnets comprises a plurality of magnets (116); and
the magnetic element (110) further comprises an electrically conductive paste (174) interposed between one or more of the plurality of magnets (116).

4. The catheter (100) of any preceding claim, wherein:
the second electrode wire distal end (152) is coupled to a proximally facing surface of the magnetic element (110); and
a first electrode wire proximal end (144) is coupled to a distally facing surface of the magnetic element (110).

5. The catheter (100) of claim 4, wherein the second electrode wire distal end (152) is frayed such that the second electrode wire distal end (152) comprises a plurality of contacting wires, and each of the plurality of contacting wires is coupled to the proximally facing surface of the magnetic element (110).

6. The catheter (100) of claim 4, wherein the first electrode wire proximal end (144) is frayed such that the first electrode wire proximal end (144) comprises a plurality of contacting wires, and each of the plurality of contacting wires is coupled to the distally facing surface of the magnetic element (110).

7. The catheter (100) of claim 4, wherein:
the second electrode wire distal end (152), the first electrode wire proximal end (144), or both further comprise a cup-type connection (166);
the cup-type connection (166) couples to the magnetic element (110) through the use of an adhesive, an interference fit, or both; and
the cup-type connection (166) defines a first recess (167) sized and shaped to receive the proximally facing surface, distally facing surface, or both, of the magnetic element (110).

8. The catheter (100) of claim 7, wherein:
the cup-type connection (166) also defines a second recess (169) sized and shaped to receive one or more prongs (168) connected to, and extending from, the magnetic element (110); and
the one or more prongs (168) are electrically conductive.

9. The catheter (100) of claim 4, wherein:
the first electrode wire proximal end (144) comprises a conductive plate (162, 164), and the conductive plate (162, 164) is coupled to the distally facing surface of the magnetic element (110); and/or
the second electrode wire distal end (152) comprises a conductive plate (162, 164), and the conductive plate (162, 164) is coupled to the proximally facing surface of the magnetic element (110).

10. The catheter (100) of claim 9, wherein:
the conductive plate (162, 164) of the first electrode wire proximal end (144) is rectangular or circular; and/or
the conductive plate (162, 164) of the second electrode wire distal end (152) is rectangular or circular.

11. The catheter (100) of any preceding claim, wherein the magnetic element (110) is coated in a conductive coating (117).

12. The catheter (100) of any preceding claim, wherein the magnetic element (110) is coated in a thermally insulating material (118).

13. A system (10) for forming a fistula (500) between two blood vessels (400, 402), comprising:
a first catheter (100) being the catheter according to any preceding claim; and
a second catheter (200) configured to coapt with the first catheter (100) and receive the first electrode wire (140);
optionally wherein the first catheter (100) is configured to be positioned within a first blood vessel (400), and the second catheter (200) is configured to be positioned within a second blood vessel (402) adjacent to the first blood vessel (400).

14. The system (10) of claim 13, wherein the second catheter (200) comprises:
a second catheter body (202); and
a magnetic element (210) positioned along the second catheter body (202) and configured to magnetically mate with the magnetic element (110) of the first catheter (100).

15. The system (10) of claim 14, wherein the second catheter (200) comprises a second working site (232) positioned along the second catheter body (202), wherein the second working site (232) of the second catheter (200) is configured to receive the electrode.

## Patentansprüche

1. Katheter (100), umfassend:
einen Katheterkörper (102);
ein magnetisches Element (110), das entlang des Katheterkörpers (102) angeordnet ist, wobei das magnetische Element (110) ein distales Ende (112) und ein proximales Ende (114) umfasst;
einen ersten Elektrodendraht (140), der ausgebildet ist, aus einem entlang des Katheterkörpers (102) angeordneten Arbeitsbereich (132) hervorzuragen, wobei der erste Elektrodendraht (140) ein proximales Ende des ersten Elektrodendrahts (144) umfasst; und
einen zweiten Elektrodendraht (150), der ein distales Ende des zweiten Elektrodendrahts (152) umfasst, wobei:
das proximale Ende des ersten Elektrodendrahts (144) an das distale Ende (112) des magnetischen Elements (110) gekoppelt ist;
das distale Ende des zweiten Elektrodendrahts (152) an das proximale Ende (114) des magnetischen Elements (110) gekoppelt ist;
**dadurch gekennzeichnet, dass**
das magnetische Element (110) ausgebildet ist, Elektrizität von dem ersten Elektrodendraht (140) zu dem zweiten Elektrodendraht (150) zu leiten; und
der erste Elektrodendraht (140) an das distale Ende (112) des magnetischen Elements (110) gekoppelt ist und das distale Ende des zweiten Elektrodendrahts (152) an das proximale Ende (114) des magnetischen Elements (110) gekoppelt ist, um einen Stromfluss von dem zweiten Elektrodendraht (150) durch das magnetische Element (110) zu dem ersten Elektrodendraht (140) zu ermöglichen.

2. Katheter (100) nach Anspruch 1, wobei das magnetische Element (110) eine Magnetanordnung umfasst.

3. Katheter (100) nach Anspruch 2, wobei:
die Magnetanordnung eine Vielzahl von Magneten (116) umfasst; und
das magnetische Element (110) weiter eine elektrisch leitfähige Paste (174) umfasst, die zwischen einem oder mehreren der Vielzahl von Magneten (116) angeordnet ist.

4. Katheter (100) nach einem vorstehenden Anspruch, wobei:
das distale Ende des zweiten Elektrodendrahts (152) an eine proximal zugewandte Oberfläche des magnetischen Elements (110) gekoppelt ist; und
ein proximales Ende des ersten Elektrodendrahts (144) an eine distal zugewandte Oberfläche des magnetischen Elements (110) gekoppelt ist.

5. Katheter (100) nach Anspruch 4, wobei das distale Ende des zweiten Elektrodendrahts (152) so aufgespleißt ist, dass das distale Ende des zweiten Elektrodendrahts (152) eine Vielzahl von Kontaktdrähten umfasst, und jeder der Vielzahl von Kontaktdrähten an die proximal zugewandte Oberfläche des magnetischen Elements (110) gekoppelt ist.

6. Katheter (100) nach Anspruch 4, wobei das proximale Ende des ersten Elektrodendrahts (144) so aufgespleißt ist, dass das proximale Ende des ersten Elektrodendrahts (144) eine Vielzahl von Kontaktdrähten umfasst, und jeder der Vielzahl von Kontaktdrähten an die distal zugewandte Oberfläche des magnetischen Elements (110) gekoppelt ist.

7. Katheter (100) nach Anspruch 4, wobei:
das distale Ende des zweiten Elektrodendrahts (152), das proximale Ende des ersten Elektrodendrahts (144) oder beide weiter eine becherartige Verbindung (166) umfassen;
die becherartige Verbindung (166) unter Verwendung eines Klebstoffs, einer Presspassung oder beider mit dem magnetischen Element (110) gekoppelt wird; und
die becherartige Verbindung (166) eine erste Ausnehmung (167) definiert, die so dimensioniert und geformt ist, dass sie die proximal zugewandte Oberfläche, die distal zugewandte Oberfläche oder beide des magnetischen Elements (110) aufnimmt.

8. Katheter (100) nach Anspruch 7, wobei:
die becherartige Verbindung (166) auch eine zweite Ausnehmung (169) definiert, die so dimensioniert und geformt ist, dass sie einen oder mehrere Vorsprünge (168) aufnimmt, die mit dem magnetischen Element (110) verbunden sind und sich von diesem erstrecken; und
der eine oder die mehreren Vorsprünge (168) elektrisch leitfähig sind.

9. Katheter (100) nach Anspruch 4, wobei:
das proximale Ende des ersten Elektrodendrahts (144) eine leitfähige Platte (162, 164) umfasst, und die leitfähige Platte (162, 164) an die distal zugewandte Oberfläche des magnetischen Elements (110) gekoppelt ist; und/oder
das distale Ende des zweiten Elektrodendrahts (152) eine leitfähige Platte (162, 164) umfasst, und die leitfähige Platte (162, 164) an die proximal zugewandte Oberfläche des magnetischen Elements (110) gekoppelt ist.

10. Katheter (100) nach Anspruch 9, wobei:
die leitfähige Platte (162, 164) des proximalen Endes des ersten Elektrodendrahts (144) rechteckig oder kreisförmig ist; und/oder
die leitfähige Platte (162, 164) des distalen Endes des zweiten Elektrodendrahts (152) rechteckig oder kreisförmig ist.

11. Katheter (100) nach einem vorstehenden Anspruch, wobei das magnetische Element (110) mit einer leitfähigen Beschichtung (117) beschichtet ist.

12. Katheter (100) nach einem vorstehenden Anspruch, wobei das magnetische Element (110) mit einem thermisch isolierenden Material (118) beschichtet ist.

13. System (10) zum Bilden einer Fistel (500) zwischen zwei Blutgefäßen (400, 402), umfassend:
einen ersten Katheter (100), der der Katheter nach einem vorstehenden Anspruch, ist; und einen zweiten Katheter (200), der ausgebildet ist, mit dem ersten Katheter (100) zu koaptieren und den ersten Elektrodendraht (140) aufzunehmen;
wahlweise, wobei der erste Katheter (100) ausgebildet ist, innerhalb eines ersten Blutgefäßes (400) positioniert zu werden, und der zweite Katheter (200) ausgebildet ist, innerhalb eines zweiten Blutgefäßes (402), das an das erste Blutgefäß (400) angrenzt, positioniert zu werden.

14. System (10) nach Anspruch 13, wobei der zweite Katheter (200) Folgendes umfasst:
einen zweiten Katheterkörper (202); und
ein magnetisches Element (210), das entlang des zweiten Katheterkörpers (202) angeordnet ist und ausgebildet ist, mit dem magnetischen Element (110) des ersten Katheters (100) magnetisch zusammenzuwirken.

15. System (10) nach Anspruch 14, wobei der zweite Katheter (200) einen zweiten Arbeitsbereich (232) umfasst, der entlang des zweiten Katheterkörpers (202) angeordnet ist, wobei der zweite Arbeitsbereich (232) des zweiten Katheters (200) ausgebildet ist, die Elektrode aufzunehmen.

## Revendications

1. Cathéter (100) comprenant :
un corps (102) de cathéter ;
un élément magnétique (110), positionné le long du corps (102) de cathéter, l'élément magnétique (110) comprenant une extrémité distale (112) et une extrémité proximale (114) ;
un premier fil électrode (140), configuré de manière à faire saillie à partir d'un site de travail (132) positionné le long du corps (102) de cathéter, le premier fil électrode (140) comprenant une extrémité proximale (144) de premier fil électrode ; et
un deuxième fil électrode (150), comprenant une deuxième extrémité distale (152) d'électrode, dans lequel :
l'extrémité proximale (144) de premier fil électrode est reliée à l'extrémité distale (112) de l'élément magnétique (110) ;
l'extrémité distale (152) de deuxième fil électrode est reliée à l'extrémité proximale (114) de l'élément magnétique (110) ;
**caractérisé en ce que**
l'élément magnétique (110) est configuré de manière à conduire l'électricité depuis le premier fil électrode (140) jusqu'au deuxième fil électrode (150) ; et
le premier fil électrode (140) est relié à l'extrémité distale (112) de l'élément magnétique (110) et l'extrémité distale (152) de deuxième fil électrode est reliée à l'extrémité proximale (114) de l'élément magnétique (110), de manière à permettre une circulation du courant depuis le deuxième fil électrode (150), à travers l'élément magnétique (110), jusqu'au premier fil électrode (140).

2. Cathéter (100) selon la revendication 1, dans lequel l'élément magnétique (110) comprend un réseau d'aimants.

3. Cathéter (100) selon la revendication 2, dans lequel :
le réseau d'aimants comprend une pluralité d'aimants (116) ; et
l'élément magnétique (110) comprend en outre une pâte électriquement conductrice (174) interposée entre un ou plusieurs aimants de la pluralité d'aimants (116).

4. Cathéter (100) selon une quelconque revendication précédente, dans lequel :
l'extrémité distale (152) de deuxième fil électrode est reliée à une surface tournée vers le côté proximal de l'élément magnétique (110) ; et
une extrémité proximale (144) de premier fil électrode est reliée à une surface tournée vers le côté distal de l'élément magnétique (110).

5. Cathéter (100) selon la revendication 4, dans lequel l'extrémité distale (152) de deuxième fil électrode est effilochée de sorte que l'extrémité distale (152) de deuxième fil électrode comprend une pluralité de fils de contact, et chacun de la pluralité des fils de contact est relié à la surface tournée vers le côté proximal de l'élément magnétique (110).

6. Cathéter (100) selon la revendication 4, dans lequel l'extrémité proximale (144) de premier fil électrode est effilochée de sorte que l'extrémité proximale (144) de premier fil électrode comprend une pluralité de fils de contact, et chacun de la pluralité des fils de contact est relié à la surface tournée vers le côté distal de l'élément magnétique (110).

7. Cathéter (100) selon la revendication 4, dans lequel :
l'extrémité distale (152) de deuxième fil électrode ou l'extrémité proximale (144) de premier fil électrode, ou les deux, comprennent en outre une connexion de type coupelle (166) ;
la connexion de type coupelle (166) est reliée à l'élément magnétique (110) par l'utilisation d'un adhésif, d'un ajustement serré, ou des deux ; et
la connexion de type coupelle (166) définit un premier évidement (167) dimensionné et façonné pour recevoir la surface tournée vers le côté proximal, la surface tournée vers le côté distal, ou les deux, de l'élément magnétique (110).

8. Cathéter (100) selon la revendication 7, dans lequel :
la connexion de type coupelle (166) définit également un deuxième évidement (169) dimensionné et façonné pour recevoir un ou plusieurs ergots (168) reliés à l'élément magnétique (110), et faisant saillie à partir de celui-ci ; et
lesdits un ou plusieurs ergots (168) sont électriquement conducteurs.

9. Cathéter (100) selon la revendication 4, dans lequel :
l'extrémité proximale (144) de premier fil électrode comprend une plaque conductrice (162, 164), et la plaque conductrice (162, 164) est reliée à la surface tournée vers le côté distal de l'élément magnétique (110) ; et/ou
l'extrémité distale (152) de deuxième fil électrode comprend une plaque conductrice (162, 164), et la plaque conductrice (162, 164) est reliée à la surface tournée vers le côté proximal de l'élément magnétique (110).

10. Cathéter (100) selon la revendication 9, dans lequel :
la plaque conductrice (162, 164) de l'extrémité proximale (144) de premier fil électrode est rectangulaire ou circulaire ; et/ou
la plaque conductrice (162, 164) de l'extrémité distale (152) de deuxième fil électrode est rectangulaire ou circulaire.

11. Cathéter (100) selon une quelconque revendication précédente, dans lequel l'élément magnétique (110) est revêtu d'un revêtement électriquement conducteur (117).

12. Cathéter (100) selon une quelconque revendication précédente, dans lequel l'élément magnétique (110) est revêtu d'un matériau thermiquement isolant (118).

13. Système (10) de formation d'une fistule (500) entre deux vaisseaux sanguins (400, 402), comprenant :
un premier cathéter (100) étant le cathéter selon une quelconque revendication précédente ; et
un deuxième cathéter (200) configuré de manière à coapter avec le premier cathéter (100) et à recevoir le premier fil électrode (140) ;
dans lequel, facultativement, le premier cathéter (100) est configuré de manière à être positionné à l'intérieur d'un premier vaisseau sanguin (400), et le deuxième cathéter (200) est configuré de manière à être positionné à l'intérieur d'un deuxième vaisseau sanguin (402) adjacent au premier vaisseau sanguin (400).

14. Système (10) selon la revendication 13, dans lequel le deuxième cathéter (200) comprend :
un deuxième corps de cathéter (202) ; et
un élément magnétique (210) positionné le long du deuxième corps de cathéter (202) et configuré de manière à s'associer magnétiquement à l'élément magnétique (110) du premier cathéter (100).

15. Système (10) selon la revendication 14, dans lequel le deuxième cathéter (200) comprend un deuxième site de travail (232) positionné le long du deuxième corps de cathéter (202), dans lequel le deuxième site de travail (232) du deuxième cathéter (200) est configuré de manière à recevoir l'électrode.
